(19) Europäisches Patentamt
European Patent Office
Office européen des brevets

(11) **EP 2 712 366 B2**

(12) **NEW EUROPEAN PATENT SPECIFICATION**

After opposition procedure

(45) Date of publication and mention
of the opposition decision:
**11.11.2020 Bulletin 2020/46**

(45) Mention of the grant of the patent:
**11.10.2017 Bulletin 2017/41**

(21) Application number: **12768239.1**

(22) Date of filing: **27.03.2012**

(51) Int Cl.:
*A61L 9/04* *(2006.01)*    *C08J 9/224* *(2006.01)*
*B01D 11/02* *(2006.01)*    *C08G 63/90* *(2006.01)*
*C08J 9/12* *(2006.01)*    *C08J 9/18* *(2006.01)*
*C08J 9/232* *(2006.01)*

(86) International application number:
**PCT/SE2012/050335**

(87) International publication number:
**WO 2012/138282 (11.10.2012 Gazette 2012/41)**

(54) **METHOD OF IMPREGNATING AND PURIFYING POLYLACTIC ACID RESIN**

VERFAHREN ZUR IMPRÄGNIERUNG UND REINIGUNG EINES POLYMILCHSÄUREHARZES

PROCÉDÉ D'IMPRÉGNATION ET DE PURIFICATION DE RÉSINE DE POLY(ACIDE LACTIQUE)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.04.2011 SE 1150293**

(43) Date of publication of application:
**02.04.2014 Bulletin 2014/14**

(73) Proprietor: **Biopolymer Network Limited
Rotorua (NZ)**

(72) Inventors:
• **MARCUSSON, Anders
S-18134 Lidingö (SE)**
• **KARTHÄUSER, Joachim
S-19251 Sollentuna (SE)**
• **HAMREFORS, Jan
S-18751 Täby (SE)**

(74) Representative: **Forresters IP LLP
Skygarden
Erika-Mann-Straße 11
80636 München (DE)**

(56) References cited:
WO-A1-2008/045516    WO-A1-2008/045516
WO-A1-2008/093284    WO-A1-2008/093284
WO-A1-2010/053242    US-A- 5 049 328
US-A- 5 049 328    US-A- 5 271 886
US-A- 5 478 921    US-A1- 2005 288 485
US-A1- 2008 269 449    US-A1- 2010 029 793
US-B1- 6 180 755

• HERBERGER J ET AL: "Carbon dioxide
extraction of residual solvents in
poly(lactide-co-glycolide) microparticles",
JOURNAL OF CONTROLLED RELEASE,
ELSEVIER, AMSTERDAM, NL, vol. 90, no. 2, 24
June 2003 (2003-06-24), pages 181-195,
XP004431311, ISSN: 0168-3659, DOI:
10.1016/S0168-3659(03)00152-4
• KOEGLER WENDY S ET AL: "Carbon dioxide
extraction of residual chloroform from
biodegradable polymers", JOURNAL OF
BIOMEDICAL MATERIALS RESEARCH, WILEY,
NEW YORK, NY, US, vol. 63, no. 5, 22 August 2002
(2002-08-22), pages 567-576, XP002602824, ISSN:
0021-9304, DOI: 10.1002/JBM.10209
• KOEGLER, W.S. ET AL.: 'Carbon Dioxide
Extraction of Residual Chloroform from
Biodegradable Polymers' JOURNAL OF
BIOMEDICAL MATERIALS RESEARCH vol. 63,
no. 5, August 2002, pages 567 - 576, XP002602824
• HERBERGER, J. ET AL.: 'Carbon dioxide
extraction of residual solvents in
poly(lactide-co-glycolide) microparticles'
JOURNAL OF CONTROLLED RELEASE vol. 90,
no. 2, June 2003, pages 181 - 195, XP004431311
• Yunqing Kang et al: "Preparation,
characterization and in vitro cytotoxicity of
indomethacin-loaded PLLA/PLGA microparticles
using supercritical C02 technique", European
Journal of Pharmaceutics and
Biopharmaceutics, vol. 70 2008, pages 85-97,
• JOSEPH M. DESIMONE: "practical approaches to
green solvents", green chemistry, vol. 297, 2
August 2002 (2002-08-02), pages 799-803,

EP 2 712 366 B2

- Andrew Cooper: "Polymer synthesis and processing using supercritical carbon dioxide", the royal society of chemistry, vol. 10 2000, pages 207-234,

- Nalawade S. et al: "Supercritical carbon dioxide as a green solvent for processing polymer melts: Processing aspects and applications", PROGRESS IN POLYMER SCIENCE, vol. 31 2006, pages 19-43,

**Description**

TECHNICAL FIELD

The present invention relates to a method of impregnating a purifying polylactic acid (PLA) resin, and to the use of liquid $CO_2$ for impregnating and purifying PLA resin beads.

BACKGROUND OF THE INVENTION

There is an increasing demand for polylactic acid (PLA) resin foams for use in packaging applications, since such polymers are bio-derived and bio-degradable. These foams have good performance and are economic, and are often preferred over petroleum-derived foams such as polystyrene foam, due to increasing environmental awareness and consumer, demands.

Carbon dioxide is a useful blowing agent for PLA foams, being an environmentally friendly blowing agent without any ozone depletion potential. Some processes that use carbon dioxide as a blowing agent involve foaming and moulding, and PLA resin beads impregnated with carbon dioxide may then be used. Such processes generally involve impregnating the beads with gaseous or supercritical $CO_2$, pre-expanding the impregnated beads, resting and sometimes treating the pre-expanded beads, before re-impregnating them with more $CO_2$ or another blowing agent and further expanding and fusing them in a mould, see e.g. EP 1 378 538. Another known process involving impregnation of PLA resin beads with carbon dioxide is disclosed in WO 2008/093284. KOEGLER, W.S. et al. "Carbon Dioxide Extraction of Residual Chloroform from Biodegradable Polymers", Journal of Biomedical Materials Research, Wiley, New York, NY, US, Vol. 63, No. 5, 22 Aug. 2002, p. 567-576, ISSN: 0021-9304, DOI:10.1002/JBM.10209 discloses extraction of residual chloroform in objects of degradable polymers. US5049328A describes simultaneous blowing and purification of polymer particles, of e.g. polystyrene.

In some packaging applications, in particular in food or medical grade packaging applications, it is important that the packaging foam material does not contain any undesired substances, to avoid any possible transfer of extractives from the packaging foam to the package contents.

SUMMARY OF THE INVENTION

An object of the present invention is to provide the use of liquid $CO_2$ in a convenient method of providing PLA resin beads useful for foaming an manufacture of clean foam packages, and shock absorbing and insulating materials. The liquid $CO_2$ treatment, involving impregnation and extraction treatment may be carried out in a batch process or in a continuous process.

In particular, in a first aspect of the present invention there is provided the use of liquid $CO_2$ for impregnating and purifying PLA resin beads by subjecting PLA resin beads to a liquid $CO_2$- treatment, which involves impregnation and extraction in a method comprising

at least one impregnation and extraction step, in which the PLA resin beads are brought into contact with 0,01-1000 kg pure liquid $CO_2$/kg PLA in a reactor for a predetermined time period, at a temperature of -10 - +15 °C and pressure of 4,5-8 MPa in order to prevent the beads from sticking together and foaming, and that

a portion of the liquid $CO_2$ is drained from the PLA beads after completion of the impregnation and extraction step, at a temperature of -10 - +15 °C and pressure of 4,5-8 MPa in order to prevent the beads from sticking together and foaming, and at which the $CO_2$ is in liquid form, while leaving at least 0,01 kg liquid $CO_2$/kg PLA, preferably 0,01-1 kg liquid $CO_2$/kg PLA in the reactor containing the PLA resin beads, followed by

evacuation of $CO_2$ gas from the reactor so as to decrease the pressure to ambient pressure, whereby the remaining liquid $CO_2$ is evaporated, thereby decreasing the temperature so as to prevent foaming at ambient pressure.

The impregnation and extraction step comprises 2 or more successive steps, wherein the PLA resin beads are brought into contact with pure liquid $CO_2$ in each successive step for a predetermined time period at a temperature and pressure that prevents the beads from sticking together and foaming; and wherein at least a portion of the liquid $CO_2$ is drained from the PLA beads at the end of each step, at a temperature that prevents the beads from sticking together and foaming, and at which the $CO_2$ is in liquid form.

The impregnation and extraction of the PLA resin beads in the liquid $CO_2$-treatment, may instead comprise impregnation and extraction in a continuous process, wherein PLA resin beads are held in a reactor, in which the PLA resin beads are brought into contact with liquid $CO_2$ for a predetermined time period, at a temperature of -10 - +15 °C and pressure of 4,5-8 MPa in order to prevent the beads from sticking together and foaming, and at which the $CO_2$ is in liquid form; and pure liquid $CO_2$ is continuously fed at one end of the reactor, and is drained from another end of the reactor; and a portion of the liquid $CO_2$ is drained from the PLA beads upon completion of the impregnation and extraction, at a temperature of -10 - +15 °C and pressure of 4,5-8 MPa in order to prevent the beads from sticking together and foaming, and at which the $CO_2$ is in liquid form, while leaving at least 0,01 kg liquid $CO_2$/kg PLA, preferably 0,01-1 kg liquid $CO_2$/kg PLA in the reactor containing the PLA resin beads, said reactor being dimensioned so that the time of contact between the PLA resin beads and the liquid $CO_2$ is sufficient to achieve desired purity of the PLA beads; followed by evacuation of $CO_2$ gas from the reactor so as to decrease the pressure to ambient pressure, whereby the remaining liquid $CO_2$ is evaporated, thereby decreasing the temperature so as to prevent uncontrolled foaming.

The liquid $CO_2$-treatment involving impregnation and extraction may be a part of a method of manufacturing PLA foam, wherein $CO_2$ functions as a blowing agent.

The impregnation and extraction is performed at a pressure of 4,5-8 MPa. The extraction is performed at a temperature of -10 - +15 °C, and below the glass transition temperature of PLA at the $CO_2$ pressure used. For economical reasons the total amount of liquid $CO_2$ may be in the range of 2-20 kg $CO_2$ / kg PLA.

The liquid $CO_2$ drained from the impregnation and extraction is preferably transferred to a recovery equipment, in which the $CO_2$ is recovered by separation of extractives from the $CO_2$ by distillation. The recovered $CO_2$ may then be recycled and reused in further impregnation and extraction of PLA resin beads. Further, the liquid $CO_2$ may contain additives chosen from coloring agents, anti-static agents, flame retardants, adhesive, perfume, or pigments are impregnated or coated on to the PLA resin beads during the impregnation and extraction.

DRAWINGS

Figure 1 shows a schematic view of a system, useful for performing impregnation and purification of PLA resin beads.
Figure 2 shows a schematic view of a system, similar to the one of Fig. 1, and which includes a pump.

DETAILED DESCRIPTION

The use of liquid $CO_2$ in the method of impregnating and purifying polylactic acid (PLA) of the present invention allows for manufacture of expandable PLA resin having reduced content of extractives, and is a convenient and simple process that allows the impregnated unfoamed beads to be stored and/or transported, before being processed further.

In the present context the term "extractables" refers to substances that are extracted from PLA during impregnation with carbon dioxide.

The term "extractives" refers to substances that can be extracted from PLA with diethyl ether as extraction medium according to the method of determination of extractives in PLA as described in the description of this patent application.

Additives or coating materials can be added to the PLA resin beads during impregnation and extraction. Accordingly, by means of the present invention various functionalities can effectively be added to the material, since impregnation, extraction and addition of additives can be performed in one process, without any need for additional process steps.

Polylactic acid or PLA is a polymer or copolymer comprising lactic acid monomer units. For the purposes of the present invention references to polylactic acid includes crystalline and amorphous polymers and mixtures thereof. The PLA resin beads may comprise about 50-100 % by weight of PLA. The PLA may comprise amorphous PLA or a blend of amorphous PLA and crystalline PLA. Preferred blends comprise at least about 50 % by weight of amorphous PLA and about 0-50 % by weight of crystalline PLA. The lactic acid in the resin beads may comprise one or more lactic acid isomers including L-lactic acid, D-lactic acid or DL-lactic acid. The lactic acid is preferably L-lactic acid. The PLA may be produced industrially by polymerisation of lactic acid obtained by the bacterial fermentation of biomass such as beet, sugarcane, cornstarch or milk products.

Commercially available PLA resin beads can be impregnated without any pretreatment using liquid $CO_2$. Amorphous, crystalline, and amorphous-crystalline blends may be used but results with highly crystalline grades are sometimes of lesser quality at the preferred pressure and temperature ranges. Reference to resin "beads" is intended to mean the crude resin material (often in the form of pellets) obtained from manufacturers and the terms beads, granules and pellets may be used interchangeably. The beads may also be in the form of fibres. Beads may be resized by extrusion and pelletizing of the commercially available material using known techniques.

The present invention is based on the use of liquid $CO_2$ for the impregnation of PLA. In liquid $CO_2$ impregnation the PLA beads have little tendency to stick to each other, especially when using sub-cooled liquid $CO_2$, i.e. liquid $CO_2$ at higher pressure than equilibrium pressure at a given temperature.

Liquid $CO_2$ impregnation requires moderate temperature and pressure. The impregnated beads do not stick together and after releasing the pressure from the impregnation reactor they can be handled as a bulk commodity.

In the use of impregnating and purifying polylactic acid (PLA) according to the present disclosure PLA resin beads are subjected to a liquid $CO_2$ treatment, which involves impregnation and extraction. When carried out in a batch

process, the liquid $CO_2$ treatment comprises at least one impregnation and extraction step, in which the PLA resin beads are brought into contact with 0,01-1000 kg pure liquid $CO_2$/kg PLA in a reactor for a predetermined time period, at a temperature and pressure that prevents the beads from sticking together and foaming. During the impregnation and extraction step, $CO_2$ is impregnated into the PLA resin beads, and at the same time extractable substances are dissolved into the liquid $CO_2$. After completion of the impregnation and extraction step a portion of the liquid $CO_2$ is drained from the PLA beads, at a temperature that prevents the beads from sticking together and foaming, and at which the $CO_2$ is in liquid form, while leaving at least 0,01 kg liquid $CO_2$/kg PLA, preferably 0,01-1 kg liquid $CO_2$/kg PLA in the reactor containing the PLA resin beads. Thereafter, evacuation of $CO_2$ gas from the reactor is performed so as to decrease the pressure to ambient pressure, whereby the remaining liquid $CO_2$ is evaporated, thereby decreasing the temperature so as to prevent foaming at ambient pressure.

It has been found that liquid $CO_2$ is effective in extracting undesired substances from the PLA. By ensuring that the $CO_2$ is drained from the reactor in liquid form, the substances extracted from the PLA beads are effectively removed. By leaving a small portion of the liquid $CO_2$ in the reactor after draining it can thus be ensured that the temperature of the beads is kept low to prevent foaming when the pressure is decreased to ambient pressure at the end of the process.

The impregnation and extraction step preferably comprises 2 or more successive steps, wherein the PLA resin beads are brought into contact with pure liquid $CO_2$ in each successive step for a predetermined time period at a temperature and pressure that prevents the beads from sticking together and foaming. In the first of these impregnation and extraction steps, a part of the total impregnation is obtained. The following steps continue the impregnation and the PLA resin beads are extracted with liquid $CO_2$, each time further lowering the content of extractable substances in the PLA resin beads. At the end of each successive step at least a portion of the liquid $CO_2$ is drained from the PLA beads, at a temperature that prevents the beads from sticking together and foaming, and at which the $CO_2$ is in liquid form. By draining away the liquid $CO_2$ after each successive step, substances extracted into the liquid $CO_2$ are removed from the PLA beads, and pure liquid $CO_2$ is then added so that the next successive impregnation and extraction step can be carried out. Thus, the content of extractable undesired substances is successively reduced until a desired purity has been reached. If desired, the first impregnation and extraction step be continued until the impregnation is completed, and extraction steps are the performed subsequently. The total treatment time will then be longer than necessary from a removal-of extractives point of view, but may be desired for other reasons, such as addition of additives to the PLA.

The liquid $CO_2$-treatment, involving impregnation and extraction treatment may be carried out in a batch process or in a continuous process.

In a batch process the PLA resin beads are held in a reactor to which an amount of liquid $CO_2$ is added, and after a certain time period the liquid $CO_2$ is drained from the reactor, while substantially maintaining the pressure. A new amount of pure liquid $CO_2$ is then added to the reactor. After completion of the last impregnation and extraction step, a portion of the liquid $CO_2$ is drained from the PLA beads, and a smaller amount of liquid $CO_2$ is left in the reactor. As said above, the remaining liquid $CO_2$ is evaporated when $CO_2$ gas is evacuated from the reactor and the temperature is thereby decreased so as to prevent foaming at ambient pressure. The PLA beads may be placed in a rotating drum during the impregnation and extraction step. When gas is evacuated from the drum, the cold liquid $CO_2$ is distributed evenly in the PLA material, which gives a thorough and even cooling of the PLA beads.

When carrying out the liquid $CO_2$-treatment in a continuous process the PLA resin beads are held in a reactor, in which the PLA resin beads are brought into contact with liquid $CO_2$ for a predetermined time period, at a temperature that prevents the beads from sticking together and foaming, and at which the $CO_2$ is in liquid form. Pure liquid $CO_2$ is continuously fed at one end of the reactor, and the liquid $CO_2$ is continuously drained from another end of the reactor, so that the liquid $CO_2$ flows through the reactor. The reactor is dimensioned so that the time of contact between the PLA resin beads and the liquid $CO_2$ is sufficient to achieve the desired impregnation and extraction. Upon completion of the impregnation and extraction a portion of the liquid $CO_2$ is drained from the PLA beads, and a smaller amount of liquid $CO_2$ is left in the reactor, and is evaporated during evacuation of $CO_2$ gas from the reactor whereby the temperature is decreased. Also in a continuous process, the PLA beads may be placed in a rotating drum during the impregnation and extraction step. When gas is evacuated from the drum, the cold liquid $CO_2$ is distributed evenly in the PLA material, which gives a thorough and even cooling of the PLA beads.

The pressure and temperature during the impregnation and extraction are chosen so as to prevent the PLA resin beads from sticking together and foaming, and to provide the $CO_2$ in liquid phase. The impregnation and extraction is performed at a pressure of 4,5-8 MPa, to ensure that the $CO_2$ can be present in liquid phase in a workable temperature range, and to avoid formation of dry ice, and to provide conditions under which $CO_2$ can be in liquid phase at temperatures around 0 °C, and which leads to reasonable treatment time. The temperature of the impregnation and the extraction is -10 to +15 °C, to ensure liquid phase $CO_2$ during the treatment, and below the glass transition temperature of PLA at the $CO_2$ pressure used to avoid the PLA beads from sticking together, The temperature of the impregnation and the extraction is -10 to +15 °C, in order to achieve reasonable treatment time and ensures that $CO_2$ is liquid at the pressure chosen in the range of 4,5-8 MPa. The total amount of liquid $CO_2$ used in the liquid $CO_2$-treatment is advantageously

in the range of 2-20 kg $CO_2$ / kg PLA.

The liquid $CO_2$ drained from the impregnation and extraction may be transferred to a recovery equipment, in which the $CO_2$ is recovered by separation of extractives from the $CO_2$ by distillation. The recovered $CO_2$ may then be recycled and reused in further impregnation and extraction step of PLA resin beads. Accordingly, the $CO_2$ can be recirculated in a closed circuit, which is advantageous from both an environmental and economical point of view.

In the impregnation and extraction step the PLA resin beads are placed the reactor (a pressure vessel) under impregnation pressure and temperature, and liquid $CO_2$ is then added to the reactor until the PLA resin beads are at least partially submerged, or if a rotating drum is used, until the amount of liquid $CO_2$ is sufficient to wet the PLA beads. The PLA resin beads are left in contact with the liquid $CO_2$ for a predetermined time, preferably 10 to 240 minutes, to achieve sufficient $CO_2$ content, and at the same time avoid unnecessary treatment time in order to save costs.

A person skilled in the art will be aware that the absorption percentages, temperatures and pressures can be manipulated relative to each other at the different stages of the method of the invention to achieve substantially the same result with the major limiting factors being the preference for keeping the $CO_2$ weight percentage within or close to the optimum range of percentages, avoiding the excessive formation of dry ice in the pressure vessel, and retaining control of any foaming steps.

METHOD OF DETERMINATION OF EXTRACTIVES IN PLA

The material of the present invention is an expanded polylactic acid (PLA) resin which comprises a total amount of extractives of 0-0,035 % by weight. The total amount of extractives is calculated based on the total weight of PLA, said extractives being substances that can be extracted from PLA with diethyl ether as extraction medium.

The total amount of extractives is determined by means of the following method, which accordingly is a method used to describe the quantitative determination of extractable compounds in PLA (polylactic acid). 2.6-diethylnaphtalene (DiEN) is used as internal standard (ISTD). No correction is made to compensate for the individual extractives detector responses in relation to the internal standard. In the determination method standard solutions are prepared, said solutions being an internal standard stock solution (ISTD0) and an internal standard working solution (ISTD1).

Preparation of internal standard solution - stock solution, ISTD 0

The stock solution is prepared by dissolving 0.04 to 0.05 g of DiEN in 50 mL of acetone (99.9%). The concentration of DiEN will be approximately 0.9 g/L. The true concentration is calculated.

Preparation of internal standard solution - working solution ISTD 1

Dilute 5 mL of stock solution to 25 mL with acetone (99.9%). The concentration of DiEN will be approximately 180 mg/L. Calculate the true concentration.

Sample preparation and extraction of extractives

PLA beads are ground in a centrifugal mill (Retsch ZM1) under cooling with liquid nitrogen. The sample is ground to a size of 0.5 mm. An amount of approximately 1 g ground sample is put into a round bottom flask for reflux and 100 mL of diethyl ether is added (the true amount of PLA sample is noted). A few anti-bumping granules are put into the round bottom flask. Before starting the reflux, the sample is standing for 5 minutes. The reflux is performed for 1 hour and the diethyl ether is thereafter collected and reduced to 10 mL and 10 $\mu$L of ISTD 1 is added.

Analysis of extractives. The extractives were analysed by Thermo Finnigan Trace gas chromatograph-mass spectrometer (GC-MS)

Instrument parameters and settings

Column: Zebron zb-5MSi, 30 m, id 0.25 mm, thickness 0.25 um.
Column temperature: 80°C for 1 min and then 10°C/min until 350°C is reached and finaly, 350°C for 8 min
Carrier gas: 0.7 mL/min of helium at a constant flow
Inlet temperature: 250°C
Splittless injection for 1 minute, where the whole sample is let onto to the column. Transfer temperature (temperature between GC and MS): 320°C
Temperature in ion source: 200°C

MS mode: Total ion current (TIC)

Calculations

The concentration of extractives (Ci) in sample extract is calculated

$$Ci \ (mg/L) = Cis * (A \ i/A \ is)$$

Cis = concentration of ISTD in sample extract (mg/L)
Ai = peak area of extractives
Ais = peak area of ISTD

Amount of extractives in PLA (Cp) is calculated

$$Cp \ (mg/kg) = Ci * V / m$$

V = volume of sample extract (L)
m = dry content of PLA (kg)

EXAMPLES

The invention will now be illustrated in non-limiting ways by reference to the following examples of impregnation of PLA resin beads. In the examples below PLA beads were used without additional treatment, i.e. as commercially available.

Example 1 (Analysis of reference sample)

*PLA Ingeo 4060D™ (Nature Works LLC, USA)* as commercially available was extracted with diethyl ether according to the above described procedure for determining extractives in PLA, and analysed with GC-MS on extractable low molecular compounds. Among detected extractives, lactide was dominating followed by other lactic acid oligomers. The result is shown in Table 1.

Example 2 - liquid $CO_2$ drained off

The aim of Example 2 was to determine extractable substances extracted from the PLA, which may not have been detected by the extraction with diethylether.
*PLA Ingeo 4060D™ (Nature Works LLC, USA)* as commercially available was placed in an autoclave which was filled with liquid carbon dioxide and pressurised to 60 bars at 0-2 °C. The ratio by weight between PLA and carbon dioxide was 1/10. After four hours, liquid $CO_2$ was drained off and $CO_2$ was collected in a gas-tight bag (1L, Tedlar, SKC). The gas-tight bag was carefully cleaned and checked for background before sample collection. The $CO_2$ liquid level was allowed to decrease until the ratio by weigh between PLA and CO2 was 1/2. The remaining liquid $CO_2$ was evaporated during the evacuation of $CO_2$, thereby decreasing the temperature below Tg for $CO_2$ impregnated PLA.
Extractable substances in the gas phase was analysed according to the procedure below. Lactide was dominating followed by other lactic acid oligomers.
The followed procedure was applied for gas analysis. 5-hexen-1-ol was used as internal standard, and a working solution was prepared by dissolving approximately 1 gram of 5-hexen-1-ol in 250 mL methanol. The true concentration was calculated. From the working solution 2 µL was injected into the gas-tight bag. The bag was conditioned in room temperature for 1 hour before analysis. Solid phase microextraction (SPME) supplied with Carboxen 0,75 µm absorption phase was inserted into the gas-tight bag. After the SPME phase has been exposed to the sampled gas for 30 minutes it was transferred to the GC-MS for analysis.
Instrument parameters and settings

Column: Zebron zb-624, 30 m, id 0.25 mm, film thickness 1.40 um
Column temperature: 40°C for 4 min and then 10°C/min until 250°C followed by 250°C for 6 min
Carrier gas: 1 mL/min of helium at a constant flow
Inlet temperature: 240°C

Splittless injection for 1 minute

Transfer temperature (temperature between GC and MS): 240°C
Temperature in ion source: 200°C
MS mode: Total ion current (TIC)

Example 3 - liquid phase $CO_2$ removed by evaporation

The aim of Example 3 was to determine substances extracted from the PLA, which may not have been detected by the extraction with diethyl ether, by a method different to the one used in Example 2.

*PLA Ingeo 4060D™ (NatureWorks LLC, USA)* as commercially available was placed in an autoclave which was filled with liquid carbon dioxide (PLA/$CO_2$; 1/10 by weight) and pressurised to 50 bars at 0 °C. After 80 minutes, the liquid phase $CO_2$ was removed by evaporation and the pressure released. Substances released from PLA during impregnation were collected from the reactor chamber by extraction it with acetone, and analysed with GC-MS. Among detected extractable substances, lactide was dominating followed by other lactic acid oligomers.

Example 4 - liquid $CO_2$ drained off (1 impregnation and extraction step)

*PLA Ingeo 4060D™ (Nature Works LLC, USA)* as commercially available was placed in an autoclave and treated with liquid carbon dioxide (PLA/$CO_2$, 1/100) at 53 bars at -5-0°C for 90 minutes. The liquid phase was drained off until the ratio by weight between PLA and $CO_2$ was 1/20) and the remaining liquid $CO_2$ was evaporated during evacuation of $CO_2$ gas in order to lower the temperature below Tg for $CO_2$ impregnated PLA.

The remaining low molecular substances in the purified PLA was determined by extraction with diethyl ether according to the above described method and analysed with GC-MS. Detected substances was lactide and other lactic acid oligomers. The result is shown in Table 1.

Example 5 - liquid $CO_2$ drained off (2 impregnation and extraction steps)

PLA Ingeo 4060D™ *(NatureWorks LLC, USA)* as commercially available was placed in an autoclave and treated with liquid carbon dioxide (PLA/$CO_2$; 1/100 by weight) at 53 bars at -5-0 °C for 90 minutes. The liquid phase was drained off until the ratio by weight between PLA and $CO_2$ was 1/20). A second treatment in which pure liquid $CO_2$ was added at 53 bars was done at 53 bars, -5-0 °C for 90 minutes. The liquid phase was drained off until the ratio by weight between PLA and $CO_2$ was 1/20) and the remaining liquid $CO_2$ was evaporated during evacuation of $CO_2$ gas in order to lower the temperature below Tg for $CO_2$ impregnated PLA..

The remaining low molecular substances in the purified PLA were determined by extraction with diethyl ether according to the described method and analysed with GC-MS. Detected substances was lactide and other lactic acid oligomers. The result is shown in Table 1.

Example 6 - liquid $CO_2$ drained off (3 impregnation and extraction steps)

*PLA Ingeo 4060D™ (NatureWorks LLC, USA)* as commercially available was placed in an autoclave. PLA was treated with liquid carbon dioxide (PLA/$CO_2$, 1/100) at 53 bars, -5-0 °C for 90 minutes. The liquid phase was drained off until the ratio by weight between PLA and $CO_2$ was 1/20). The procedure was repeated with pure liquid $CO_2$ two more times. The liquid phase was drained off until the ratio by weight between PLA and $CO_2$ was 1/20) and the remaining liquid $CO_2$ was evaporated during evacuation of $CO_2$ gas in order to lower the temperature below Tg for $CO_2$ impregnated PLA. The result is shown in Table 1.

The remaining low molecular substances in the purified PLA were determined by extraction with diethyl ether according to the described method and analysed with GC-MS. Detected substances was lactide and other lactic acid oligomers.

Example 7 - liquid $CO_2$ drained off (4 impregnation and extraction steps)

*PLA Ingeo 4060D™ (NatureWorks LLC, USA)* as commercially available was placed in an autoclave. PLA was treated with liquid carbon dioxide (PLA/CO2, 1/100) at 53 bars, -5-0 °C for 90 minutes. The liquid phase was drained off until the ratio by weight between PLA and $CO_2$ was 1/20) The procedure was repeated with pure liquid $CO_2$ three times. The liquid phase was drained off until the ratio by weight between PLA and $CO_2$ was 1/20) and the remaining liquid $CO_2$ was evaporated during evacuation of $CO_2$ gas in order to lower the temperature below Tg for $CO_2$ impregnated PLA. The result is shown in Table 1.

The remaining low molecular substances in the purified PLA were determined by extraction with diethyl ether according to the described method and analysed with GC-MS. Detected substances was lactide and other lactic acid oligomers.

The remaining extractives in PLA beads after impregnation and extraction in Examples 1 and 4-7 are shown in Table 1.

Table 1

| Example | Number of impregnation and extraction steps | Total amount of extractives* % by weight | Lactide % by weight | Others* % by weight |
|---------|---------------------------------------------|------------------------------------------|---------------------|---------------------|
| 1 | 0 | 0,043 | 0,026 | 0,017 |
| 4 | 1 | 0,031 | 0,017 | 0,014 |
| 5 | 2 | 0,027 | 0,014 | 0,013 |
| 6 | 3 | 0,013 | 0,005 | 0,008 |
| 7 | 4 | 0,013 | 0,006 | 0,007 |
| * dominating substances were lactic acid oligomers. | | | | |

The results shown in Table 1 indicate that draining off the liquid $CO_2$ in liquid phase after the impregnating and extraction step, is very effective to remove extractable substances, and that repeated impregnating and extraction steps further enhances this effect.

The results in Table 1 shows that three impregnation and extraction steps may be sufficient to decrease the amount of extractives to a reasonable level, which is considerably lower than the initial levels in the PLA material.

Example 8 (Dying)

PLA Nature Works Ingeo 4060D, re-extruded to 1,5 x1,5 mm, was placed in an autoclave together with a cotton bag containing, the colouring agent 1,4-diamino anthraquinone (Sigma-Aldrich) 0,4% by weight PLA . Liquid $CO_2$ was added at 47 bars and 0-1 °C until the ratio by weight between PLA and $CO_2$ was 1/10 . After 90 minutes treatment liquid $CO_2$ was drained off until the ratio by weight between PLA and $CO_2$ was 1/2, and the remaining liquid $CO_2$ was evaporated during evacuation of $CO_2$ gas in order to lower the temperature below Tg for $CO_2$ impregnated PLA. Purple PLA pellets were collected from the reactor.

EXEMPLIFYING EMBODIMENTS

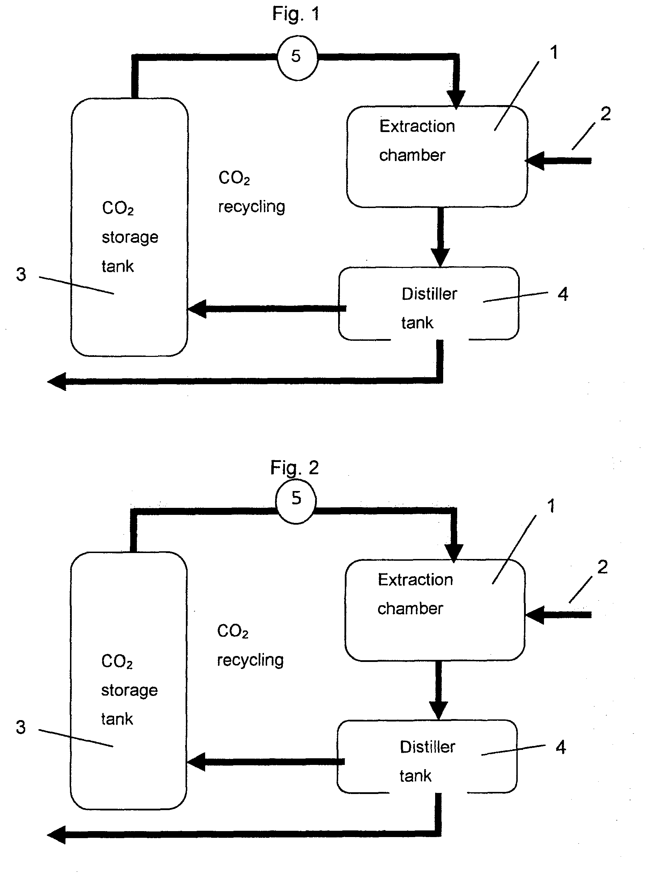

Fig 1 shows a schematic view of a system for performing a process including the method of impregnating and purifying PLA resin.

In the start-up phase of the process an extraction chamber 1 is loaded with PLA resin beads through an inlet 2. The extraction chamber may be a reactor in the form of an autoclave or any other vessel that may be pressurised. Air present in the extraction chamber 1 is evacuated by means of a vacuum pump. During start-up of the process, $CO_2$ gas is supplied to the extraction chamber 1 from a $CO_2$ storage tank 3, so as to pressurize the extraction chamber 1 to approximately 6 MPa with CO2 gas. Sensors in the system monitor the level of $CO_2$ in the storage tank 3, and additional $CO_2$ is added to the extraction chamber 1 storage tank 3_from an external source (not shown) if necessary.

In the impregnation and extraction phase, the pressures between the extraction chamber 1 and the storage tank 3 are balanced to a pressure P0. Liquid $CO_2$ is added from the storage tank 3 to the extraction chamber 1 at a pressure in the same range as P0, and the temperature T0, by means of a compressor, which sucks gas from extraction chamber 1 and pushes out liquid $CO_2$ from the bottom of the storage tank 3 by adding it to the top. If desired additives for coating or impregnating into the PLA material can be added to the liquid $CO_2$. The temperature in the extraction chamber 1 is decreased by evaporation of liquid $CO_2$ to a pressure P1 and temperature T1. $CO_2$ gas is taken from the storage tank 3 by the compressor and is supplied at the top of the extraction chamber 1 to a pressure P2 at a temperature T1, thereby achieving a subcooling of the liquid $CO_2$, thus preventing the PLA resin beads from sticking together. The temperature T1 should be below the glass transition temperature Tg of PLA for the actual $CO_2$ pressure. Evaporation and addition of $CO_2$ gas so as to achieve subcooling of the liquid $CO_2$ is repeated as often as necessary to maintain the desired conditions in the extraction chamber 1. After a predetermined treatment time period, the pressures between the extraction chamber 1 and the distiller tank 4 are balanced, and liquid $CO_2$ is drained off from the extraction chamber to the distiller tank 4. The liquid CO2 in the distiller tank 4 is distilled and transferred back to the storage tank 3. The impregnation and extraction phase may be repeated several times, as desired.

When the impregnation and extraction treatment in the liquid $CO_2$-treatment of the PLA resin beads is completed a last draining of the liquid $CO_2$ is carried out, in which the liquid $CO_2$ is partially drained off from the extraction chamber to the distiller tank 4. The remaining liquid $CO_2$ is evaporated and is transferred via a compressor via a heat exchanger/cooler to the storage tank 3. Thereby, the impregnated PLA resin beads are cooled an undesired expansion is prevented, while the pressure in the extraction chamber is lowered to atmospheric pressure. Rotation of the PLA beads during this step is preferred in order to get a uniform cooling of PLA. The impregnated PLA resin beads can then be taken out from the extraction chamber and be transferred to further processing.

Liquid $CO_2$ received in the distiller tank 4 comprises extractable substances originating from the PLA resin beads. The $CO_2$ is separated from these extractable substances by distillation, and the pure $CO_2$ thereby obtained is recycled to the storage tank 1. The extractable substances remain at the bottom of the distiller tank 4, and can be removed as a concentrate.

Alternatively, in the start-up phase of the process an extraction chamber 1 is loaded with PLA resin beads through an inlet 2. The extraction chamber may be a reactor in the form of an autoclave or any other vessel that may be pressurised. Air present in the extraction chamber 1 is evacuated by means of a vacuum pump. During start-up of the process, $CO_2$ gas is supplied to the extraction chamber 1 from a $CO_2$ storage tank 3, so as to pressurize the extraction chamber 1 to approximately 1 MPa with CO2 gas. Sensors in the system monitor the level of $CO_2$ in the storage tank 3, and additional $CO_2$ is added to the extraction chamber 1 storage tank 3_from an external source (not shown) if necessary.

In the impregnation and extraction phase, the pressures between the extraction chamber 1 and the storage tank 3 are balanced to a pressure P0. Liquid $CO_2$ is added from the storage tank 3 to the extraction chamber 1 at a pressure in the same range as P0, and the temperature T0, by means of a compressor, which sucks gas from extraction chamber 1 and pushes out liquid $CO_2$ from the bottom of the storage tank 3 by adding it to the top. A rotating drum in the extraction chamber 1 is rotating the PLA bead in the liquid CO2.

After a predetermined treatment time period, the pressures between the extraction chamber 1 and the distiller tank 4 are balanced, and liquid $CO_2$ is drained off from the extraction chamber to the distiller tank 4. The liquid CO2 in the distiller tank 4 is distilled and transferred back to the storage tank 3. The impregnation and extraction phase may be repeated several times, as desired by adding new liquid CO2 from the storage tank 3 to the extraction chamber 1. If desired additives for coating or impregnating into the PLA material can be pumped in to the extraction chamber during the process.

When the impregnation and extraction treatment in the liquid $CO_2$-treatment of the PLA resin beads is completed a last draining of the liquid $CO_2$ is carried out, in which the liquid $CO_2$ is partially d rained off from the extraction chamber to the distiller tank 4. The remaining liquid $CO_2$ is evaporated and is transferred via a compressor via a heat exchanger/cooler to the storage tank 3. Thereby, the impregnated PLA resin beads are cooled an undesired expansion is prevented, while the pressure in the extraction chamber is lowered to atmospheric pressure. Rotation of the PLA beads during this step is preferred in order to get a uniform cooling of PLA. The impregnated PLA resin beads can then be taken out from the extraction chamber and be transferred to further processing.

Liquid $CO_2$ received in the distiller tank 4 comprises extractable substances originating from the PLA resin beads. The $CO_2$ is separated from these extractable substances by distillation, and the pure $CO_2$ thereby obtained is recycled to the storage tank 1. The extractable substances remain at the bottom of the distiller tank 4, and can be removed as a concentrate.

The above system relates to a batch process. However, the process may as well be performed continuously, and the system is then adapted thereto.

Fig 2 shows a schematic view of a system for performing a process including the method of impregnating and purifying PLA resin. In the start-up phase of the process an extraction chamber 1 is loaded with PLA resin beads through an inlet 2. The extraction chamber may be a reactor in the form of an autoclave or any other vessel that may be pressurised. Air present in the extraction chamber 1 is evacuated by means of a vacuum pump. During start-up of the process, $CO_2$ gas is supplied to the extraction chamber 1 from a $CO_2$ storage tank 3, so as to pressurize the extraction chamber 1 to approximately 6 MPa with $CO_2$ gas. Sensors in the system monitor the level of $CO_2$ in the storage tank 3, and additional $CO_2$ is added to the extraction chamber 1 from an external source if necessary.

In the impregnation and extraction phase, the pressures between the extraction chamber 1 and the storage tank 3 are balanced to a pressure P0. Liquid $CO_2$ is added from the storage tank 3 to the extraction chamber 1 using a liquid $CO_2$ pump 5. The setpoint temperature is maintained using cooling unit. The pump is used to maintain the setpoint pressure in the extraction chamber during the extraction/impregnation. The PLA particles are rotated in a drum in the extraction chamber 1. The $CO_2$ in the extraction chamber 1 is drained to the distiller tank 4 after a predetermined time. New $CO_2$ is added from the storage tank 3 to the extraction chamber 1 while $CO_2$ in the distiller tank 4 is distilled and transported to storage tank 3 while extraction/impregnation of PLA occurs in the extraction chamber 1 in the same way as in the first bath. The number of $CO_2$ baths can vary depending on purity level of the PLA. In any of the baths an additive can be added to the extraction chamber 1 to modify the properties of the PLA.

When the liquid $CO_2$-treatment, which involves impregnation and extraction of the PLA resin beads is completed a last draining of the liquid $CO_2$ is carried out, in which the liquid $CO_2$ is partially drained off from the extraction chamber to the distiller tank 4. The remaining liquid $CO_2$ is evaporated and is transferred via a compressor via a heat exchanger/cooler to the storage tank 3. Thereby, the impregnated PLA resin beads are cooled an undesired expansion is prevented, while the pressure in the extraction chamber is lowered to atmospheric pressure. Rotation of the PLA beads during this step is preferred in order to get a uniform cooling of PLA. The impregnated PLA resin beads can then be taken out from the extraction chamber and be transferred to further processing.

## Claims

1. Use of liquid $CO_2$ for impregnating and purifying PLA resin beads by subjecting PLA resin beads to a liquid $CO_2$-treatment, which involves impregnation and extraction in a method comprising

   at least one impregnation and extraction step, in which the PLA resin beads are brought into contact with 0,01-1000 kg pure liquid $CO_2$ /kg PLA in a reactor for a predetermined time period, at a temperature of -10 - +15 °C and pressure of 4,5-8 MPa in order to prevent the beads from sticking together and foaming, and that

   a portion of the liquid $CO_2$ is drained from the PLA beads after completion of the impregnation and extraction step, at a temperature of -10 - +15 °C and pressure of 4,5-8 MPa in order to prevent the beads from sticking together and foaming, and at which the $CO_2$ is in liquid form, while leaving at least 0,01 kg liquid $CO_2$/kg PLA, preferably 0,01-1 kg liquid $CO_2$ /kg PLA in the reactor containing the PLA resin beads, followed by

   evacuation of $CO_2$ gas from the reactor so as to decrease the pressure to ambient pressure, whereby the remaining liquid $CO_2$ is evaporated, thereby decreasing the temperature so as to prevent foaming at ambient pressure.

## Patentansprüche

1. Verwendung von flüssigem $CO_2$ für die Imprägnierung und Reinigung von PLA-Harzkugeln durch das Unterwerfen der PLA-Harzkugeln einer Behandlung mit flüssigem $CO_2$, welche die Imprägnierung und Extraktion umfasst, in einem Verfahren umfassend

   - mindestens einen Imprägnierungs- und Extraktionsschritt, bei welchem die PLA-Harzkugeln in einem Reaktor für einen vorgegebenen Zeitraum, bei einer Temperatur von -10 - +15 °C und einem Druck von 4,5-8 MPa, mit 0,01-1000 kg von reinem, flüssigem $CO_2$/kg PLA in Kontakt gebracht werden, um das Zusammenkleben und die Schaumbildung der Kuglen zu verhindern, und
   - ein Teil des flüssigen CO2 wird nach der Durchführung von jedem Imprägnierungs- und Extraktionsschritt, bei einer Temperatur von -10 - +15 °C und einem Druck von 4,5-8 MPa, von den PLA-Kugeln entzogen, um das Zusammenkleben und die Schaumbildung der Kugeln zu verhindern, und bei welcher sich das $CO_2$ in flüssiger Form befindet, während zumindest 0,01 kg von flüssigem $CO_2$/kg PLA, vorzugsweise 0,01-1 kg von flüssigem $CO_2$/kg PLA, in dem die PLA-Harzkugeln enthaltenden Reaktor zurückbehalten werden, und anschließend
   - Evakuierung von $CO_2$-Gas von dem Reaktor, um den Druck auf Umgebungsdruck zu senken, wobei das verbleibende flüssige $CO_2$ verdampft wird, wodurch die Temperatur gesenkt wird, um eine Schaumbildung bei Umgebungsdruck zu verhindern.

## Revendications

1. Usage de $CO_2$ liquide pour l'imprégnation et l'extraction de billes de résine PLA par la soumission des billes de résine PLA à un traitement de $CO_2$ liquide, qui implique l'imprégnation et l'extraction dans un procédé comprenant au moins une étape d'imprégnation et d'extraction, dans laquelle les billes de résine PLA sont mises en contact avec 0,01-1000 kg de $CO_2$ pur et liquide dans un réacteur pendant une période de temps prédéterminée, à une température de -10 - +15 °C et à une pression de 4,5-8 MPa, afin d'empêcher les billes de se coller et de mousser, et qu'une partie du $CO_2$ liquide est vidangée à partir des billes PLA après la fin de chaque étape d'imprégnation et d'extraction, à une température de -10 - +15 °C et à une pression de 4,5-8 MPa afin d'empêcher les billes de se coller et de mousser, et à laquelle le $CO_2$ est sous forme liquide, tout en laissant au moins 0,01 kg $CO_2$/kg PLA liquide, de préférence 0,01-1 kg $CO_2$/kg PLA liquide dans le réacteur contenant les billes de résine PLA, suivi par l'évacuation du gaz $CO_2$ depuis le réacteur afin de diminuer la pression à la pression ambiante, si bien que le $CO_2$

liquide restant est évaporé en diminuant ainsi la température afin d'éviter le moussage à la pression ambiante.

## Fig. 1

## Fig. 2

## REFERENCES CITED IN THE DESCRIPTION

**Patent documents cited in the description**

- EP 1378538 A
- WO 2008093284 A
- US 5049328 A

**Non-patent literature cited in the description**

- Carbon Dioxide Extraction of Residual Chloroform from Biodegradable Polymers. **KOEGLER, W.S. et al.** Journal of Biomedical Materials Research. Wiley, 22 August 2002, vol. 63, 567-576